# EUROPÄISCHE PATENTANMELDUNG

(11) **EP 2 946 776 A1**
(43) Veröffentlichungstag der Anmeldung: **25.11.2015**
(21) Anmeldenummer: 14169039.6
(22) Anmeldetag: 20.05.2014
(51) Int. Cl.: A61K 9/70, A61K 31/55, A61P 25/06

(54) **Transdermales therapeutisches System zur Abgabe von Amitriptylin**

(71) Anmelder: LTS LOHMANN Therapie-Systeme AG, 56626 Andernach (DE)
(72) Erfinder: Weiler, Erika, D-56316 Niederhofen (DE); Laux, Wolfgang, D-65582 Diez (DE)
(74) Vertreter: Schweitzer, Klaus

(57) **Zusammenfassung**

Ein transdermales therapeutisches System in Pflasterform zur Verabreichung des Wirkstoffs Amitriptylin weist eine wirkstoffundurchlässige Rückschicht, ein damit verbundenes, den Wirkstoff Amitriptylin enthaltendes Wirkstoffreservoir, eine hautseitige haftklebende Schicht und eine vor der Applikation ablösbare wirkstoffundurchlässige Schutzschicht auf. Es dient zur Behandlung und Prophylaxe der Migräne oder des Spannungskopfschmerzes.

## Beschreibung

Die vorliegende Erfindung betrifft transdermale therapeutische Systeme mit dem Wirkstoff Amitriptylin, welche die Verabreichung dieses Wirkstoffs über die Haut ermöglichen. Die Erfindung umfasst ferner die therapeutische Verwendung derartiger Systeme bei der Behandlung und der Prophylaxe der Migräne und des Spannungskopfschmerzes.

Amitriptylin ist ein Arzneistoff aus der Gruppe der trizyklischen Antidepressiva, der in erster Linie zur Behandlung von Depressionen und zur langfristigen Schmerzbehandlung eingesetzt wird. Auf Grund seiner nachgewiesenen migräneprophylaktischen Wirkung gilt Amitriptylin als Mittel der ersten oder zweiten Wahl bei der Prophylaxe der Migräne. Auch zur vorbeugenden Behandlung des Spannungskopfschmerzes gilt Amitriptylin als der am besten untersuchte Arzneistoffe.

Die orale Applikation von Amitriptylin ist allerdings mit einer Reihe von Nachteilen verbunden, die durch die Pharmakokinetik bedingt sind. Nach erfolgter oraler Verabreichung treten in der Anfangsphase "Peak-Plasma-Spitzen" auf, d. h. die Amitriptylin-Plasmakonzentration erreicht kurzzeitig relativ hohe Werte. Dies kann bei einer üblichen Tagesdosis von 10 - 25 mg zu unerwünschten Nebenwirkungen führen.

Bei der prophylaktischen Behandlung der Migränes durch orale Verabreichung von Amitriptylin wurden u.a. schon folgende Nebenwirkungen beobachtet: Müdigkeit, Schwindel, verminderte Konzentration, Appetitlosigkeit, Gewichtszunahme, Haarausfall, Veränderungen des Libido und Benommenheit.

Die Halbwertszeit von Amitriptylin im Körper beträgt 8 - 51 Stunden; die Halbwertzeit der Amitriptylin-Metaboliten 30 Stunden. Der Metabolit Nortriptylin ist ebenfalls wirkaktiv.

Transdermale therapeutische Systeme (TTS) sind Darreichungsformen, die auf die Haut appliziert werden und einen Wirkstoff an die Haut abgeben, wobei dieser Wirkstoff dadurch systemisch verfügbar gemacht wird.

TTS bestehen nach dem Stand der Technik aus einer arzneistoffundurchlässigen Trägerschicht (auch Rückschicht genannt), einer arzneistoffhaltigen Reservoirschicht sowie einer Haftklebeschicht zur Befestigung auf der Haut. Die letztgenannte Schicht kann auch mit der arzneistoffhaltigen Schicht identisch sein. Außerdem weisen TTS in der Regel eine vor der Applikation zu entfernende, ebenfalls wirkstoffundurchlässige Rückschicht auf. Zusätzlich können noch andere Komponenten vorhanden sein, wie z. B. eine die Wirkstoffabgabe begrenzende Steuermembran. Die arzneistoffhaltige Reservoirschicht besteht meist aus polymeren Grundsubstanzen; sie kann ferner verschiedene Hilfs- oder Zusatzstoffe enthalten.

Aufgabe der vorliegenden Erfindung war es, eine Darreichungsform für den Wirkstoff Amitriptylin bereitzustellen, bei deren Anwendung die Nachteile vermieden werden, die mit der oralen Verabreichung von Amitriptylin einher gehen, wie oben beschrieben.

Ferner sollte diese Darreichungsform einen genügend hohen Wirkstoff-Flux in vivo ermöglichen.

Des weiteren ist zu fordern, dass eine solche Darreichungsform mittels gängiger Herstellungsverfahren kostengünstig produziert werden kann.

Überraschenderweise wird diese Aufgabe gelöst durch ein transdermales therapeutisches System in Pflasterform gemäss Anspruch 1, sowie durch die in den Unteransprüchen beschriebenen besonderen Ausführungsformen.

Folglich umfasst die Erfindung TTS in Pflasterform zur Verabreichung des Wirkstoffs Amitriptylin, welche eine wirkstoffundurchlässige Rückschicht, ein damit verbundenes, den Wirkstoff Amitriptylin oder dessen pharmazeutisch akzeptable Säureadditionssalz, vorzugsweise dessen Hydrochlorid enthaltendes Wirkstoffreservoir, eine hautseitige haftklebende Schicht und eine vor der Applikation ablösbare wirkstoffundurchlässige Schutzschicht aufweisen.

Die erfindungsgemäßen TTS ermöglichen über einen verlängerten Applikations-Zeitraum eine konstante Abgabe des Wirkstoffs Amitriptylin an und durch die Haut, wodurch dieser Wirkstoff systemisch verfügbar wird. Auf diese Weise kann die relativ rasche Elimination von Amitriptylin durch ständiges Nachliefern aus dem Wirkstoffreservoir des TTS kompensiert werden, und der therapeutische Wert der Arzneistoffverabreichung wird erhöht.

Dies ist insbesondere bei der Behandlung und der Prophylaxe der Migräne und des Spannungskopfschmerzes vorteilhaft. Gegenüber oralen Darreichungsformen lässt sich eine fortgesetzte therapeutische Wirkung mit einer relativ niedrigen Applikationsfrequenz erzielen.

Weitere Vorteile beruhen darauf, dass bei der transdermalen Verabreichung die Metabolisierung des Wirkstoffs während der ersten Darm-Leber-Passage ("First-Pass"-Effekt) verhindert wird und damit die Halbwertszeit erhöht wird. Außerdem können auf diese Weise Probleme wie gastrointestinale Intoleranz oder unzureichende enterale Absorption vermieden werden.

Die erfindungsgemäßen TTS können sowohl in Form von Matrix-Systemen als auch in Form von Beutel-Reservoir- bzw. Membransystemen hergestellt werden.

Der Begriff "Matrixsysteme" schließt nicht nur solche Systeme ein, bei denen der Wirkstoff in einer schichtförmigen Kunstharz- oder Kunststoffmatrix gelöst ist und aus dieser abgegeben wird, sondern auch solche, in denen der Wirkstoff an Fasermaterial, wie z. B. Baumwollgewebe oder Baumwollvlies adsorbiert ist. Dieses Fasermaterial kann in einer Kunststoff- oder Kunstharzmatrix eingebettet sein.

Grundsätzlich können für die Herstellung des Wirkstoffreservoirs bzw. der Wirkstoffmatrix eine Vielzahl von Polymeren, Harzen und Zusatzstoffen eingesetzt werden, sofern die mit der Haut in Berührung kommenden Stoffe hautverträglich sind und solange die damit hergestellte Formulierung in der Lage ist, den Wirkstoff Amitriptylin an die Haut abzugeben.

Das Wirkstoffreservoir der erfindungsgemäßen TTS kann ferner verschiedene Hilfs-oder Zusatzstoffe enthalten, beispielsweise aus der Gruppe der Lösungsvermittler, Lösungsmittel, Weichmacher, Permeationsverbesserer, pH-Regulatoren, Antioxidantien und Konservierungsmittel.

Bei der Herstellung der erfindungsgemäßen TTS kann im einfachsten Fall so vorgegangen werden, dass Amitriptylin in einer Lösung von Matrix-Grundpolymeren grob, kolloidal oder molekular dispergiert wird und die Mischung auf eine geeignete Unterlage, beispielsweise eine mit einer Silikonschicht versehene thermoplastische Folie (Release Liner), beschichtet wird.

Nach Trocknen und Abdampfen der Lösemittelanteile wird die wirkstoffhaltige Matrixschicht mit einer weiteren Folie (Backing Layer) abgedeckt. Falls Matrixschicht, Release Liner und Backing Layer transparent sind, wird ein transparentes TTS erhalten.

Durch Stanzen flächiger Gebilde in der gewünschten geometrischen Form und Größe werden aus einem solchen Laminat-TTS hergestellt.

Das TTS kann Sollbruchlinien oder Perforationen ausgestattet werden und damit portionierbar sein. Oder auf einem Release-Liner können sich mehrere TTS mit jeweils einem eigenen Wirkstoffreservoir befinden. Der Patient kann dann - je nach Intensität eines Migräneanfalls - zwei oder mehrere vorportionierte Abschnitte vom Release-Liner abziehen und sich aufkleben. Ähnliche Systeme sind beispielsweise in der EP 418608 A1 beschrieben.

Geeignete Grundpolymere für die Wirkstoffmatrix und die haftklebende Schicht und der Aufbau eines TTS sind beispielsweise in Tan, Pfister, PSTT Vol. 2, No.2 Februar 1999, Seiten 60 - 69 beschrieben. Geeignete Grundpolymere sind beispielsweise Polyacrylate, Poly(meth)acrylate, Polyacrylsäure, Cellulose-Derivate, insbesondere Methyl- und Ethylcellulosen, Polyisobutylen, Ethylen-Vinylacetat-Copolymere, natürliche und synthetische Kautschuke wie Styrol-Dien-Copolymere, Styrol-Butadien-Blockcopolymere, Styrol-Isopren-Blockcopolymere, Acrylnitril-Butadien-Kautschuk, Butylkautschuk oder Neoprenkautschuk, sowie Heißschmelzkleber. Als Haftkleber kommen auch solche auf Silikonbasis in Betracht. Mit Vorteil können auch geeignete Mischungen der genannten Polymere oder Hybridkleber aus AcrylatMonomeren und Silikon-Monomeren zum Einsatz kommen.

Weiterhin sind auch Mikroreservoir-Systeme, wie sie in der WO 01/01967 beschrieben werden, und Systeme mit zwei nebeneinander liegenden wirkstoffhaltigen Abschnitten (vgl. WO 01/26705) geeignet.

Unter den Begriff "Heißschmelzkleber" fallen alle Kleber, die nicht durch Lösemittel, sondern durch Schmelzen bei erhöhten Temperaturen, beispielsweise im Bereich von 60 - 200 °C verflüssigt werden. Als Heißschmelzkleber eignen sich z.B. Mischungen aus Estern des hydrierten Kolophoniums mit Cellulosederivaten.

Neben den genannten Polymeren können auch weitere, dem Fachmann bekannte Polymere als Grundpolymere für die Herstellung der Matrix oder der Haftklebeschicht verwendet werden, vorausgesetzt, diese sind mit dem Wirkstoff Amitriptylin kompatibel.

Eine besonders bevorzugte Ausführungsform der Erfindung ist dadurch gekennzeichnet, dass der Wirkstoff Amitriptylin im TTS in Kombination mit einem

Lösungsvermittler vorliegt, vorzugsweise in gelöstem Zustand; auch ein Gemisch von Lösungsvermittlern kann verwendet werden.

Beispiele für bevorzugte Lösungsvermittler sind mehrwertige Alkohole wie 1,2-Propandiol, die verschiedenen Butandiole, Glycerin, Polyethylenglykol 400, Tetrahydrofurfurylalkohol, Diethylenglykolmonoethylether, Diethyltoluamid und Monoisopropyliden-glycerin. Besonders bevorzugt wird 1,2-Propandiol verwendet. Einige der genannten Lösungsvermittler, wie z. B. auch das 1,2-Propandiol, können zusätzlich auch permeationsfördernd wirken.

Als vorteilhaft hat sich herausgestellt, dass der Anteil des/der Lösungsvermittler zwischen 1 und 50 Gew.-%, vorzugsweise zwischen 5 und 35 Gew.-% beträgt, bezogen auf das gesamte TTS im Endzustand nach der Herstellung.

Um einen hohen Wirkstoff-Flux durch die Haut zu erreichen, hat es sich insbesondere bei Matrix-Systemen als besonders vorteilhaft erwiesen, wenn der Matrix ein oder mehrere permeationsfördernde Stoffe zugesetzt werden, und zwar in einem Mengenanteil von 0,1 bis 25 Gew.-%, vorzugsweise von 1 bis 10 Gew.-%, jeweils bezogen auf das Gesamtgewicht der Wirkstoffmatrix.

Als permeationsfördernde Stoffe eignen sich vor allem Stoffe aus den Gruppen der Fettalkohole, Fettsäuren, Polyoxyethylenfettalkoholether, Polyoxyethylenfettsäureester, Fettalkoholester und Fettsäureester, insbesondere Sorbitanmonolaurat oder Ester von langkettigen Fettsäuren mit Methyl-, Ethyl- oder Isopropylalkohol, oder Ester von Fettalkoholen mit Essigsäure oder Milchsäure. Auch Stoffe wie Ölsäurediethanolamin kommen in Betracht. Besonders bevorzugt werden Polyoxylaurylether (Brij) eingesetzt.

Die Wirkstoffmatrix der erfindungsgemäßen TTS kann gemäss einer besonderen Ausführungsform auch einen zwei- oder mehrschichtigen Aufbau aufweisen, d. h. sie kann aus zwei oder mehreren Matrixschichten bestehen. Dabei können sich die verschiedenen Matrixschichten hinsichtlich ihrer Zusammensetzung oder der Konzentration der darin enthaltenen Bestandteile unterscheiden. Beispielsweise können die verschiedenen Matrixschichten eine unterschiedliche Polymerzusammensetzung aufweisen oder aus unterschiedlichen Haftklebern bestehen. Ferner können die einzelnen Matrixschichten unterschiedliche Konzentrationen an Wirkstoff oder Zusatzstoffen wie permeationsfördernden Mitteln, Lösungsvermittlern und Weichmachern enthalten. In Abhängigkeit von dem beabsichtigten Anwendungszweck können beispielsweise die Konzentrationen dieser Inhaltsstoffe, insbesondere die Wirkstoffkonzentration, in diesen Schichten so eingestellt werden, dass sie von der inneren Schicht zu der hautseitig gelegenen Schicht kleiner oder größer werden, je nachdem, ob eine besondere Langzeitwirkung oder eine besonders starke Initialwirkung angestrebt wird.

Bei weiteren Ausführungsformen der erfindungsgemäßen TTS ist vorgesehen, dass der Wirkstoffmatrix, oder einzelnen Schichten dieser Matrix, übliche Weichmacher in einer Konzentration von bis zu 30 Gew.-% zugesetzt werden, besonders bevorzugt in einer Konzentration von 5 - 20 Gew.-%, jeweils bezogen auf die Wirkstoffmatrix.

Als Weichmacher können bevorzugt solche aus der Gruppe der Kohlenwasserstoffe, Alkohole, Carbonsäuren, Derivate von Carbonsäuren, Ether, Ester und Amine verwendet werden.

Um eine hohe Freisetzungsrate zu erzielen, wird bevorzugt, eine möglichst hohe Wirkstoffkonzentration in der/den wirkstoffhaltigen Schicht(en) vorzusehen, wobei allerdings zu beachten ist, dass zu hohe Wirkstoffkonzentrationen die physikalische Stabilität der Wirkstoffmatrix beeinträchtigen können. Bei den erfindungsgemäßen TTS werden deshalb Wirkstoffkonzentrationen im Bereich von 0,1 bis 50 Gew.-%, bevorzugt im Bereich von 5 bis 25 Gew.-%, insbesondere im Bereich von 1 bis 10 Gew.-% angewandt, jeweils bezogen auf die Gesamtmasse der wirkstoffhaltigen Schicht(en).

Der Wirkstoffgehalt eines Eintages-TTS liegt bei 5 bis 40 mg, vorzugsweise 10 bis 30 mg. Man erhöht ihn entsprechend, wenn man sich ein 2- oder 3-Tages-TTS wünscht.

Um eine Steuerung der Wirkstofffreisetzung zu ermöglichen - sofern dies nicht durch andere Mechanismen bewirkt wird -, kann das Wirkstoffreservoir auf der Abgabeseite auch mit einer Steuermembran versehen werden, welche eine begrenzte Durchlässigkeit für den Wirkstoff aufweist und die Abgabe des Wirkstoffs an die Haut steuert.

Die haftklebende Befestigung der erfindungsgemäßen TTS auf der Haut kann auf verschiedene Arten erfolgen. Beispielsweise kann die wirkstoffhaltige Matrix selbst aus einem Haftkleber bestehen und so die Verbindung zur Haut bewirken, oder es ist eine separate haftklebende Schicht angebracht, welche diese Funktion übernimmt. Insbesondere bei Systemen, welche - wie vorstehend beschrieben - mit einer hautseitigen, nicht klebenden Steuermembran ausgestattet sind, ist es vorteilhaft, die Befestigung auf der Haut durch einen Klebstoffrand zu bewirken, der die Membranfläche, über welche der Wirkstoff an die Haut abgegeben wird, umgibt, jedoch nicht berührt; d. h. der Klebstoffrand steht mit der Steuermembran in keiner Verbindung.

Die Erfindung umfasst ferner Ausführungsformen, bei denen das Amitriptylin enthaltende Wirkstoffreservoir als beutelförmiges Reservoir gestaltet ist, welches mit einer fließfähigen, hochviskosen, halbfesten oder gelartigen Matrix gefüllt ist, die den Wirkstoff enthält. Beispielsweise kann es sich dabei um eine Polymermatrix, insbesondere um eine Kunststoffmatrix oder eine Lösung davon handeln. Besonders vorteilhaft ist es, wenn das Wirkstoffreservoir einen Gelbildner enthält.

Die der Haut abgewandte Beutelrückseite muss dabei wirkstoffundurchlässig, die der Haut zugewandte Seite (Abgabeseite) wirkstoffdurchlässig sein. Falls erforderlich, kann eine wirkstoffdurchlässige Membran die Steuerung der Wirkstofffreisetzung übernehmen. Geeignete Materialien für die Herstellung der Beutelwand bzw. der Steuermembran sind dem Fachmann bekannt.

Die erfindungsgemäßen TTS weisen neben dem Wirkstoffreservoir außerdem eine wirkstoffundurchlässige Rückschicht sowie eine ebenfalls wirkstoffundurchlässige ablösbare Schutzschicht oder Abziehfolie auf.

Als Materialien für die Rückschicht eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Ethylen-Vinylacetat-Copolymere, Polyvinylacetat, Polyethylen, Polypropylen, Polyurethane, Cellulosederivate und viele andere mehr. Im Einzelfall kann die Rückschicht mit einer zusätzlichen Auflage versehen werden, z. B. durch Bedampfung mit Metallen, insbesondere Aluminium. Zur Herstellung eines transparenten TTS verwendet man eine transparente Rückschicht.

Für die ablösbare Schutzschicht können grundsätzlich dieselben Materialien verwendet werden wie für die Rückschicht, vorausgesetzt, dass sie durch eine geeignete Oberflächenbehandlung, wie z. B. Silikonisierung, ablösbar ausgerüstet ist. Es können aber auch andere ablösbaren Schutzschichten wie z. B. mit Polytetrafluorethylen behandeltes Papier oder ^{®}Cellophan (Cellulosehydrat) verwendet werden.

Die erfindungsgemäßen TTS mit dem Wirkstoff Amitriptylin eignen sich in vorteilhafter Weise zur Behandlung und Prophylaxe der Migräne und des Spannungskopfschmerzes. Besonders vorteilhaft ist dabei, dass mit diesen Darreichungsformen eine Wirkungsdauer von mindestens etwa einem Tag bis zu etwa 3 7 Tagen erreicht werden kann, abhängig vom Wirkstoff-Gehalt und der Ausgestaltung des Steuerungsmechanismus, der das Wirkstoffabgabeverhalten kontrolliert.

Da Amitriptylin einen gewissen lokalanästhetischen Effekt hat, kann man einen lokalen Zusatzeffekt erzielen, wenn man das TTS am Kopf, im Nacken auf der Schläfe oder auf der Stirn - also in regionaler Nähe zum systemischen Zielort-platziert.

Gemäss einer weiteren Ausführungsform der Erfindung ist vorgesehen, dass das Wirkstoffreservoir Amitriptylin in Kombination mit mindestens einem weiteren pharmazeutischen Wirkstoff enthält; vorzugsweise handelt es sich dabei um einen zentral wirksamen Arzneistoff.

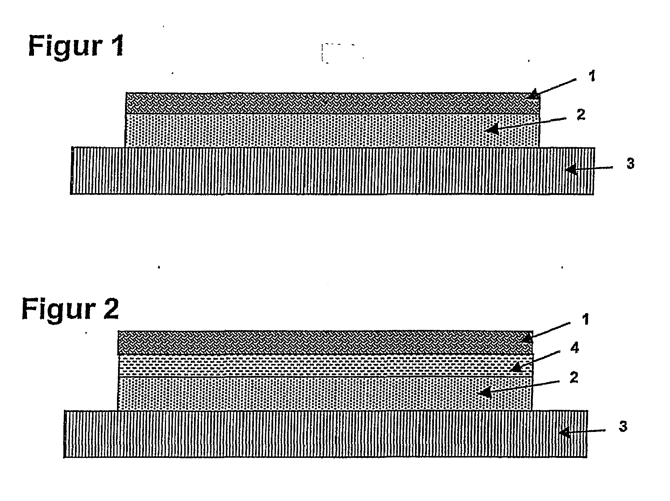

Der Aufbau der erfindungsgemäßen TTS wir anhand der Fig. 1 und Fig. 2 beispielhaft erläutert. Es handelt sich dabei um schematische, vereinfachte Zeichnungen, welche jeweils ein TTS in der Schnittdarstellung zeigen.
Fig. 1 zeigt ein TTS, bestehend aus einer Rückschicht (1) und einer homogenen, wirkstoffhaltigen Matrixschicht (2). Die hautzugewandte Seite der Matrixschicht (2) ist mit einer wiederablösbaren Schutzschicht (3) bedeckt. Dieser bevorzugte Aufbau entspricht demjenigen, der in den Beispielen beschrieben ist.
Fig. 2 zeigt eine weitere Ausführungsform eines erfindungsgemäßen TTS, die ebenfalls eine Rückschicht (1), eine homogene, wirkstoffhaltige Matrixschicht (2) und eine wiederablösbare Schutzschicht (3) aufweist. Zusätzlich befindet sich zwischen Matrixschicht (2) und Rückschicht (1) eine wasserdampfsperrende Zwischenschicht (4).

Die Erfindung wird weiterhin durch nachfolgende Beispiele erläutert ohne dass sie darauf beschränkt wäre.

### Beispiel 1

Die erfindungsgemäßen TTS können beispielsweise wie folgt hergestellt werden:

Es werden 50 g Amitriptylin sowie 20 g eines geeigneten permeationsfördernden Stoffes (z.B. ^{®}Brij 30) in 200 g 1,2-Propandiol gelöst. Diese Lösung wird mit einer geeigneten Rührapparatur in einen Silikonkleber (Nr. 4301; Fa. Dow Corning, USA) gegeben und dispergiert, so dass eine möglichst homogene Flüssig-Flüssig-Dispersion entsteht. Diese Dispersion wird mit einer geeigneten Vorrichtung homogen auf eine Trägerfolie, z. B. aus Polyethylenterephthalat beschichtet. Anschließend wird durch eine kontrollierte Trocknung das Lösungsmittel des Silikonklebers sowie etwaige Anteile des Propandiols entfernt. Kontrollierte Trocknung bedeutet, dass das beschichtete Laminat einer ganz bestimmen Trocknungstemperatur, Trocknungsgeschwindigkeit oder Trocknungszeit ausgesetzt wird, um den beabsichtigten Gehalt von flüchtigen Substanzen (z. B. Lösungsvermittler) einzustellen.

Das so erhaltene Laminat wird anschließend mit einer weiteren Folie aus Polyethylenterephthalat zukaschiert. Zuletzt werden TTS mit einer bestimmten Fläche ausgestanzt und in ein geeignetes Packmittel verpackt.

### Beispiel 2

Es werden 157 g Polyisobutylen, relative mittlere Molmasse ca. 1.270.000 (z. B. Oppanol ® B 100), Lösung (21,3 % g/g) in Benzin, 34 g Polyisobutylen, relative mittlere Molmasse ca. 40.000 (z. B. Oppanol ® B 10), 17 g Polyisobutylen, relative mittlere Molmasse ca. 800 (z. B. Oppanol ® B 3), 17 g thermoplastisches Kohlenwasserstoffharz (z. B. Escorez ® 5300) in 111 g n-Hexan unter Rühren gelöst. Zu 60 g dieser Lösung werden in einem zylindrischen Glasbehälter 1 g Amitriptylin gegeben und die Lösung mit einem Magnetrührer bis zur Auflösung gerührt. Die Lösung wird auf silikonisierte, 200 µm dicke Polyesterfolie in einer Schichtdicke von 300 µm ausgestrichen. Es wird vorsichtig getrocknet.

Das so erhaltene Laminat wird anschließend mit einer weiteren Folie aus Polyethylenterephthalat zukaschiert. Zuletzt werden TTS mit einer bestimmten Fläche ausgestanzt und in ein geeignetes Packmittel verpackt.

### Beispiel 3

8,9 g Amitriptylin. 105 g Acrylatklebstoff (Durotak ® 387-2287), 46 g klebstoffharz (z. B. Hercolyn ® DE), 10 g Eutragit ® L100, 30 g Glycerin, 15 g Acetylaceton und 0,1 g Aluminiumacetylacetonat (4 Gew.-% in Ethylacetat) werden gemischt. Die Lösung wird mit einer Nassschichtdicke von 200 µm mit Hilfe einer Rakel auf eine silikonisierte Polyesterfolie (z. B. Hostaphan ® RN) aufgetragen. Der feuchte Film wird getrocknet.

Das so erhaltene Laminat wird anschließend mit einer weiteren Folie aus Polyethylenterephthalat zukaschiert. Zuletzt werden TTS mit einer bestimmten Fläche ausgestanzt und in ein geeignetes Packmittel verpackt.

## Patentansprüche

1. Transdermales therapeutisches System in Pflasterform zur Verabreichung des Wirkstoffs Amitriptylin, welches eine wirkstoffundurchlässige Rückschicht, ein damit verbundenes, den Wirkstoff Amitriptylin oder dessen pharmazeutisch akzeptable Säureadditionssalz enthaltendes Wirkstoffreservoir, eine hautseitige haftklebende Schicht und eine vor der Applikation ablösbare wirkstoffundurchlässige Schutzschicht aufweist.

2. Transdermales therapeutisches System nach Anspruch 1, **dadurch gekennzeichnet, dass** das Wirkstoffreservoir als wirkstoffhaltige Matrix ausgebildet ist, wobei die genannte Matrix eine Kunstharz- oder Kunststoffmatrix ist, die als Grundpolymer(e) ein oder mehrere Polymere enthält, welche vorzugsweise aus der Polyacrylate, Poly(meth)acrylate, Polyacrylsäure, Cellulose-Derivate, Isobutylen, Ethylen Vinylacetat, natürliche und synthetische Kautschuke wie Styrol-Dien-Copolymere, Styrol-Butadien-Blockcopolymere, Isopren-Blockcopolymere, Acrylnitril-Butadien-Kautschuk, Butylkautschuk oder Neoprenkautschuk, sowie Heissschmelzkleber umfassenden Gruppe ausgewählt sind.

3. Transdermales therapeutisches System nach Anspruch 1 oder 2, **dadurch gekennzeichnet, dass** das Wirkstoffreservoir als wirkstoffhaltige Matrix ausgebildet ist, in welcher der Wirkstoff Amitriptylin an einem Fasermaterial, bevorzugt an Baumwollgewebe oder -vlies adsorbiert vorliegt, wobei das genannte Fasermaterial vorzugsweise in eine Kunststoff- oder Kunstharzmatrix eingebettet ist.

4. Transdermales therapeutisches System nach Anspruch 1, **dadurch gekennzeichnet, dass** das Wirkstoffreservoir als beutelförmiges Reservoir ausgebildet ist, welches eine hoch viskose oder halbfeste Kunststoffmatrix oder eine Lösung davon oder einen Gelbildner oder ein Gel enthält, worin der Wirkstoff Amitriptylin gelöst oder dispergiert ist.

5. Transdermales therapeutisches System nach einem oder mehreren der Ansprüche 1 bis 4, **dadurch gekennzeichnet, dass** das Wirkstoffreservoir hautseitig mit einer wirkstoffdurchlässigen oder einer die Wirkstoffabgaberate beschränkenden Steuermembran ausgestattet ist.

6. Transdermales therapeutisches System nach Anspruch 5, **dadurch gekennzeichnet, dass** es einen an den Außenrand der Membranfläche angrenzenden, auf der Haut haftklebenden Klebstoffrand besitzt, der nicht mit der Steuermembran in Verbindung steht.

7. Transdermales therapeutisches System nach einem oder mehreren der vorangehenden Ansprüche, **dadurch gekennzeichnet, dass** die haftklebende Schicht als Grundpolymere Polymere enthält, welche vorzugsweise aus der Polyacrylate, Poly(meth)acrylate, Polyacrylsäure, Cellulose-Derivate, Polyisobutylen, Ethylen-Vinylacetat-Copolymere, natürliche und synthetische Kautschuke wie Styrol-Dien-Copolymere, Styrol-Butadien-Blockcopolymere, Styrol-Isopren-Blockcopolymere, Acrylnitril-Butadien-Kautschuk, Butylkautschuk oder Neoprenkautschuk, sowie Heißschmelzkleber und Haftkleber auf Silikonbasis umfassenden Gruppe ausgewählt sind.

8. Transdermales therapeutisches System nach einem oder mehreren der vorangehenden Ansprüche, **dadurch gekennzeichnet, dass** das Wirkstoffreservoir als haftklebende Schicht ausgebildet ist.

9. Transdermales therapeutisches System nach einem oder mehreren der vorangehenden Anspruche, **dadurch gekennzeichnet, dass** das Wirkstoffreservoir und/oder die haftklebende Schicht mindestens einen Hilfs-oder Zusatzstoff enthält, welcher aus der Lösemittel, Lösungsvermittler, Weichmacher, Permeationsverbesserer, pH-Regulatoren, Antioxidantien und Konservierungsmittel umfassenden Gruppe ausgewählt ist.

10. Transdermales therapeutisches System nach einem oder mehreren der vorangehenden Ansprüche, **dadurch gekennzeichnet, dass** es den Wirkstoff Amitriptylin in Kombination mit einem Lösungsvermittler, besonders bevorzugt einem Lösungsvermittler mit permeationsfördernder Wirkung, enthält, wobei der Wirkstoff vorzugsweise in gelöster Form vorliegt.

11. Transdermales therapeutisches System nach einem oder mehreren der vorangehenden Ansprüche, **dadurch gekennzeichnet, dass** es einen oder mehrere Lösungsvermittler enthält, vorzugsweise ausgewählt aus der mehrwertige Alkohole, 1,2-Propandiol, Butandiole, Glycerin, Polyethylenglykol 400, Tetrahydrofurfurylalkohol, Diethylenglykolmonoethylether, Diethyltoluamid und Monoisopropyliden-glycerin umfassenden Gruppe, wobei die Konzentration des/der Lösungsvermittlers zwischen 1 und 50 Gew.-%, vorzugsweise zwischen 5 und 35 Gew.-% beträgt, bezogen auf das gesamte System.

12. Transdermales therapeutisches System nach einem oder mehreren der vorangehenden Ansprüche, **dadurch gekennzeichnet, dass** es einen oder mehrere permeationsfördernde Stoffe enthält, vorzugsweise in einer Konzentration von 0,1 bis 25 Gew.-%, besonders bevorzugt in einer Konzentration von 1 bis 10 Gew.-%, jeweils bezogen auf das gesamte System.

13. Transdermales therapeutisches System nach Anspruch 12, **dadurch gekennzeichnet, dass** der/die permeationsfördernde(n) Stoff(e) aus der Fettalkohole, Fettsäuren, Polyoxyethylenfettalkoholether, Polyoxyethylenfettsäureester, Fettsäureester und Fettalkoholester umfassenden Gruppe ausgewählt ist.

14. Transdermales therapeutisches System nach einem oder mehreren der vorangehenden Ansprüche, **dadurch gekennzeichnet, dass** die Wirkstoffmatrix einen oder mehrere Weichmacher enthält, vorzugsweise in einer Konzentration von bis zu 30 Gew.-%, besonders bevorzugt in einer Konzentration von 5 - 20 Gew.-%, jeweils bezogen auf die Wirkstoffmatrix, wobei die Weichmacher vorzugsweise aus der Kohlenwasserstoffe, Alkohole, Carbonsäuren, Derivate von Carbonsäuren, Ether, Ester und Amine umfassenden Gruppe ausgewählt sind.

15. Transdermales therapeutisches System nach einem oder mehreren der vorangehenden Ansprüche, **dadurch gekennzeichnet, dass** das Wirkstoffreservoir einen schichtförmigen Aufbau mit zwei oder mehreren Matrixschichten aufweist, die sich hinsichtlich ihres Polymergehalts und/oder ihres Wirkstoffgehalts und/oder hinsichtlich der Art und Konzentration der darin enthaltenen Permeationsverbesserer, Weichmacher oder Lösungsvermittler unterscheiden.

16. Transdermales therapeutisches System nach einem oder mehreren der vorangehenden Ansprüche, **dadurch gekennzeichnet, dass** die Wirkstoffkonzentration im Bereich von 0,1 bis 50 Gew.-%, bevorzugt im Bereich von 5 bis 25 Gew.-%, insbesondere im Bereich von 1 bis 10 Gew.-% liegt, jeweils bezogen auf die Gesamtmasse der wirkstoffhaltigen Schicht(en).

17. Transdermales therapeutisches System nach einem oder mehreren der vorangehenden Ansprüche, **dadurch gekennzeichnet, dass** das Wirkstoffreservoir neben Amitriptylin mindestens einen weiteren Wirkstoff enthält, vorzugsweise einen zentral wirksamen Wirkstoff.

18. Verwendung eines den Wirkstoff Amitriptylin enthaltenden transdermalen therapeutischen Systems zur Behandlung und Prophylaxe der Migräne oder des Spannungskopfschmerzes.

19. Amitriptylin zur Verwendung bei der Behandlung und der Prophylaxe der Migräne oder des Spannungskopfschmerzes, bei welcher man ein transdermales therapeutisches System in Pflasterform, welches eine wirkstoffundurchlässige Rückschicht, ein damit verbundenes, den Wirkstoff Amitriptylin enthaltendes Wirkstoffreservoir und eine hautseitige haftklebende Schicht aufweist, appliziert.
